Europäisches Patentamt

**European Patent Office**

**Office européen des brevets**

(19)

(11) Numéro de publication: **0 119 919**
A1

(12)

# DEMANDE DE BREVET EUROPEEN

(21) Numéro de dépôt: **84400511.6**

(22) Date de dépôt: **13.03.84**

(51) Int. Cl.³: **A 61 F 5/44,** A 61 F 13/18

(30) Priorité: **14.03.83 FR 8304170**

(43) Date de publication de la demande: **26.09.84**
**Bulletin 84/39**

(84) Etats contractants désignés: **AT BE CH DE GB IT LI LU NL SE**

(71) Demandeur: **LABORATOIRES BIOTROL, 1, rue du Foin, F-75140 Paris Cedex 03 (FR)**

(72) Inventeur: **Maupetit, Philippe, 23, rue François Massé, F-93600 Aulnay-Sous-Bois (FR)**

(74) Mandataire: **Phélip, Bruno et al, c/o Cabinet Harlé & Phélip 21, rue de la Rochefoucauld, F-75009 Paris (FR)**

(54) **Serviette absorbante pour incontinence urinaire féminine.**

(57) La présente invention concerne une serviette absorbante pour incontinence urinaire féminine.

La serviette est constituée d'une succession de couches, la couche absorbante étant surmontée d'une couche imperméable percée d'un évidement et isolée de la couche inférieure par une couche totalement imperméable, des couches supérieures poreuses répartissant le liquide. Ces couches sont solidaires.

ACTORUM AG

1.

" Serviette absorbante pour incontinence urinaire
féminine "

La présente invention concerne une serviette absorbante pour incontinence urinaire féminine.

On sait que nombre de femmes souffrent
d'un syndrome d'incontinence urinaire de différents
types. Ce peut être de l'incontinence par miction
impérieuse, où il y a émission d'urine sans envie
d'uriner préalable, ou l'incontinence d'effort, dûe
à la perte d'efficacité des moyens d'occlusions vésicales ou encore de l'incontinence permanente, où
la perte d'urine est continue. A la connaissance de
la demanderesse, aucun matériel spécifique pour recueillir ces pertes intempestives d'urines n'est à
la disposition des femmes. Sont utilisés des appareillages divers, mal adaptés, ou créés dans d'autres buts; ainsi, les garnitures périodiques, parfois certes présentées comme utilisables lors d'incontinences urinaires, ou encore les couches pour
bébé ou adulte, ou même des sondes urinaires introduites par les voies naturelles et reliées à un récipient collecteur. Ces produits sont l'objet d'un
certain nombre de brevets tant français qu'étrangers et, par exemple : FR-A-1 445 658 et 2 499 371,
GB-A-1 559 275, JA-A-2 498 447, US-A-2 496 456,
2 479 684, 2 437 842, 2 350 828, 2 248 017 et US-A-
3 665 920 et Belge 893 697.

Tous ces dispositifs, mal adaptés, ont
divers inconvénients non négligeables ; la nécessité de changer fréquemment les serviettes hygiéniques
classiques de faible capacité et de faible pouvoir
de rétention sous l'effet de la pression d'un liquide très fluide, sans compter les conséquences d'une

2.

vitesse d'absorption trop lente de l'urine, liquide non visqueux comme le sang menstruel; ou encore les problèmes esthétiques dûs au volume des couches qui interdisent aux femmes de porter d'autres vêtements qu'amples; ou encore les risques d'infection des voies urinaires lors de l'emploi de sondes, des voies uro-génitales dûs à la présence d'un corps étranger non stérile et d'une longue macération au contact de l'urine, ou plus simplement la gêne et même le risque de blessures ou d'irritation lors des mouvements (marche) dûs à ce que les dispositifs connus ne s'adaptent pas au mieux à la morphologie de toutes les incontinences urinaires, étant donné la variation importante du rapport périnéo-vulvaire d'une femme à l'autre.

Le dispositif selon la présente invention est particulièrement avantageux puisqu'il ne présente aucun des inconvénients précédemment cités, notamment du fait de la présence d'une couche non-absorbante sur la couche absorbante facilitant la répartition du liquide sur toute la surface de cette dernière, malgré une zone d'émission limitée.

Cette serviette est caractérisée par des constituants qui en font un élément d'absorption très efficace, pour un faible volume, ce qui la rend discrète, et par une forme adaptée à l'anatomie féminine. Sa structure élimine au mieux les fuites de liquide sous l'effet de la pression, et les effets de macération.

Un autre aspect avantageux de l'invention est que cette serviette, par sa structure et le choix convenable du produit absorbant qu'elle contient est utilisable pour les 3 types d'incontinence urinaire, avec le même intérêt, bien que les volumes

3.

et débits urinaires soient bien différents suivant le type d'incontinence.

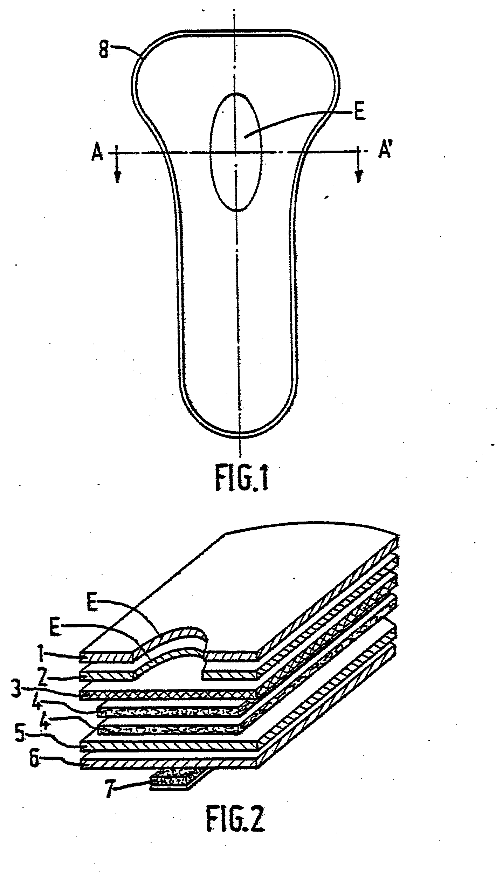

Afin de mieux faire comprendre l'invention, on a représenté Fig. 1 la serviette dans son ensemble et Fig. 2 une coupe transversale éclatée de celle-ci.

La serviette a une forme étudiée pour pouvoir se glisser entre les cuisses de la patiente et s'appliquer sur la région pubienne et pour que la plus grande partie de l'urine soit absorbée hors des zones de compression maximum, dûes au poids et au mouvement du corps de la patiente. Cette forme est allongée, pouvant aller du rectangle plus ou moins échancré, jusqu'au triangle avec des angles arrondis pour éviter irritation de la peau et gêne de la patiente. Les dimensions de la serviette sont variables, mais doivent permettre qu'elle ne dépasse pas la zone du périnée de la patiente pour que les fesses ne reposent pas dessus quand la patiente est assise, et d'autre part qu'il ne se forme pas de pliure entre les cuisses. La longueur sera d'environ 150 à 300 mm, la plus grande largeur de 50 à 200 mm, tandis que dans la partie étroite, la largeur sera de 40 à 100 mm.

Cette serviette est constituée d'une succession d'au moins 6 couches de matériau dont la nature et les propriétés sont détaillées ci-après. Un évidement de forme ovale peut être réalisé dans la couche supérieure à l'aplomb de celui existant dans la seconde et à un endroit de la serviette tel qu'il se trouve sur la zone du méat urinaire de la femme lorsque la serviette est en place.

La première couche /⁻(1) - Fig. 2_7, destinée à être au contact du corps de la patiente est

4.

en tissu non tissé, ayant subi ou non un traitement le rendant hydrofuge et pouvant être ou non adhésive du côté interne de la serviette pour le rendre solidaire de la couche suivante. Cette couche est donc évidée (E, fig. 2) sauf quand on utilise un tissu non tissé, très poreux laissant passer les liquides sans s'en imprégner, comme par exemple le non tissé 100 % BEMBERG® à 18 g/m$^2$, cellulose régénérée par un sel cupro-ammoniacal, commercialisé par Dupont de Nemours.

La seconde couche $\lceil (2)$ - fig. 2$\rfloor$ est constituée d'un film plastique imperméable aux liquides toujours évidée suivant un ovale (E - Fig. 2), un endroit tel que la découpe se trouve lorsque la serviette est correctement positionnée, dans la zone du méat urinaire de la femme. Ce film empêche le reflux de tout liquide, même sous des pressions élevées.

Ce film plastique peut être de nature très variée tel qu'en polyéthylène, polypropylène, chlorure de polyvinyle, complexe polyéthylène + chlorure de polyvinylidène + EVA.

Si la couche supérieure n'est pas adhésivée, le film plastique peut ou non être sur sa face au contact du non tissé. Il peut même être adhésivé sur les deux faces ou sur la face inférieure pour le rendre aussi solidaire de la troisième couche.

Les première et deuxième couches peuvent aussi être confondues si on utilise non plus la succession tissu non tissé et film plastique, mais un tissu composite réalisé par couchage d'un film plastique, par exemple en polyéthylène et d'un tissu non tissé. Il est bien évident que dans ce cas, la couche est toujours évidée.

7231 EU

5.

L'évidement que comporte la deuxième couche et parfois la première, a des dimensions qui lui permettent de s'adapter à toutes les formes de méat urinaire, et qui sont fonction des matériaux composant la serviette; la perforation sera ainsi plus ou moins allongée et importante. En outre, la zone périorificielle peut être munie d'un adhésif pour rendre les deux premières couches solidaires si elles ne le sont pas par un autre moyen.

La troisième couche $\underline{/}$ (3) - fig. 2 $\underline{7}$ est constituée d'un film non tissé poreux limitant la remontée du liquide par la zone d'évidement du film imperméable, et répartissant l'urine entrante sur toute la surface absorbante sous-jacente. La nature et le grammage des fibres de ce non-tissé peuvent être très variés : le polyester, la rayonne ou leur mélange conviennent et des grammages de 20 g/m$^2$ à 75 g/m$^2$ conviennent et par exemple 20, 37, 59, 72.

Une quatrième couche $\underline{/}$ (4) - Fig. 2 $\underline{7}$ constituée d'un matériau absorbant de structure spéciale à grande capacité et grande vitesse d'absorption. Cette surface absorbante a un très grand pouvoir de rétention des fluides sous l'effet de la pression et présente une vitesse très élevée de propagation de l'humidité.

Le matériau sera un absorbant classique, comme les pâtes de bois, ou un super absorbant, à base de carboxyméthylcellulose de sodium insolubilisée, à base de polyacrylates ou autres matériaux synthétiques ou naturels traités.

Les super-absorbants seront utilisés - sous forme de poudre noyée dans une pulpe liante, dans une structure formant des cannelures ou des réseaux de cellules ouverts (en nid d'abeille) - sous

7231 EU

6.

forme de feuilles de non-tissé ou de papier dans lequel il est emprisonné par un gaufrage ou laminage à chaud, tels par exemple le SAPSHEET ® de Sanyo Chemical Industries Ltd, le PERMASORB SHEET LAMINATE ® de National Starch and Chemical Corporation.

Une cinquième couche /⁻(5) - Fig. 2_7, film plastique imperméable qui empêche la diffusion de liquide dans la couche sous-jacente et constitué, comme le film de la deuxième couche, sans évidement.

Une sixième couche /⁻(6) - Fig. 2_7 en tissu non tissé formant la partie externe inférieure de la serviette qui peut être constituée, comme le film de la couche supérieure mais bien évidemment sans perforation. A cela on peut rajouter sous la sixième couche, une partie adhésive permettant la fixation de la serviette au sous-vêtement. On peut utiliser un adhésif double face dont un côté est collé sur la couche inférieure de la serviette et dont l'autre est protégé par un papier siliconé, enlevé au moment de la fixation sur le sous-vêtement. Des produits antidérapants déposés par zone sur un axe de la serviette peuvent aussi être utilisés. L'un ou l'autre pourra être déposé en continu ou non sur l'axe longitudinal de la serviette, ou encore en bandes transversales.

L'assemblage des diverses couches sera réalisé par soudure périphérique soit à la chaleur, soit aux ultra-sons, ou encore par collage à l'aide d'un ruban adhésif, d'un adhésif de transfert ou Hot-Melt /⁻(8) - figure 1_7 ou tout autre méthode appropriée en fonction des matériaux choisis.

En général, on ne fixera pas la couche absorbante aux autres de telle sorte que lorsqu'elle se gorge d'urine et se dilate, elle ne fasse pas

7.

traction avec risque de séparation des couches. Avantageusement, on ne fixera pas non plus la couche
intermédiaire entre la couche absorbante et la couche imperméable supérieure, pour éviter les fuites
de l'urine au niveau de la zone d'assemblage et
pour favoriser le passage de l'urine à la périphérie
de la couche absorbante.

Dans ce qui suit, 2 exemples de serviette
absorbante selon l'invention sont décrits :

Exemple 1.

La forme générale est celle représentée
figure 1; ce modèle a de 200 à 250 mm de long, de
100 à 130 mm de large dans sa partie supérieure, et
de 60 à 80 mm dans la zone plus étroite. L'épaisseur de la serviette terminée est inférieure à 10 mm
mais pourra aller jusqu'à 25 mm lorsqu'elle sera
gorgée d'urine.

L'évidement est un disque de 35 à 45 mm de
diamètre ou une ellipse de diamètres 30 à 40 mm et
65 à 75 mm. Son centre se trouve de 70 à 80 mm du
bord supérieur de la serviette. Le tissu non tissé
utilisé pour les première et sixième couches est
100 % polyester, à 41 g/m$^2$, de style Flannel (marque
déposée), traité hydrofuge.

Les films plastiques $\underline{/}$(2) et (5)$\underline{/}$ sont
en polyéthylène de 20 µm d'épaisseur, ce qui assure
une grande légèreté et une grande souplesse. Ils
sont non adhésifs, mais rendus solidaires des deux
tissus non tissés (1) et (6) par un couchage préalable, la couche absorbante 4 est composée d'un tissu
empli de super-absorbant à base de carboxyméthylcellulose de sodium insolubilisé, ayant 200 à 400 g/m$^2$
de super-absorbant (fabricant : Enka BV product
group industrial colloïds).

8.

L'assemblage est réalisé en soudant par ultra-sons les couches à l'exclusion de la couche absorbante, /¯c'est-à-dire (1) + (2) + (3) + (5) + (6), fig. 2_7 sur une largeur de 2 à 4 mm en laissant une bordure périphérique libre de 2 à 4 mm /¯(8) - fig. 1_7. Dans une variante, la couche non tissée (3) n'est pas prise dans cette soudure. La couche de super-absorbant est emprisonnée dans les autres de telle sorte que lors de sa dilatation par absorption d'urine, elle ne fasse pas traction sur les films soudés.

Enfin, un adhésif /¯(7) - fig. 2_7 du type de ceux qui se trouvent couramment sur les serviettes périodiques, est fixé à la face inférieure.

Exemple 2.

La forme générale est celle représentée figure 1; ce modèle a de 200 à 250 mm de long, de 100 à 130 mm de large dans sa partie supérieure, et de 60 à 80 mm dans la zone plus étroite. L'épaisseur de la serviette terminée est inférieure à 10 mm mais pourra aller jusqu'à 25 mm lorsqu'elle sera gorgée d'urine.

L'évidement est un disque de 35 à 45 mm de diamètre ou une ellipse de diamètres 30 à 40 mm et 65 à 75 mm. Son centre se trouve de 70 à 80 mm du bord supérieur de la serviette. Le tissu non tissé utilisé pour les première et sixième couches est du 100 % BEMBERG ® à 18 g/m$^2$, et la première couche n'est pas perforée en son centre.

Les films plastiques /¯(2) et (5)_7 sont en polyéthylène de 20 µm d'épaisseur, ce qui assure une grande légèreté et une grande souplesse. Ils sont non adhésifs, mais rendus solidaires des deux tissus non tissés (1) et (6) par un couchage préa-

9.

lable, la couche absorbante 4 est composée d'un tissu empli de super-absorbant à base de carboxyméthylcellulose de sodium insolubilisé, ayant 200 à 400 g/m$^2$ de super-absorbant (fabricant : Enka BV product group industrial colloïds).

L'assemblage est réalisé par collage à la périphérie des couches, mais à une distance de 2 à 4 mm, pour les couches [(1) + (2) + (5) + (6) fig. 2], c'est-à-dire en laissant libres la couche absorbante et la couche en BEMBERG® .

La surface extérieure de la couche 6 est totalement enduite d'un produit antidérapant couramment utilisé dans la technique.

7231 EU

10

## REVENDICATIONS

1.Serviette absorbante pour incontinence urinaire féminine,caractérisée en ce qu'elle est peu épaisse,de forme adaptée à l'anatomie féminine et qu'elle est constituée de différentes couches,de telle sorte que la couche absorbante soit surmontée d'une couche non-absorbante facilitant la répartition du liquide dans toute sa surface, elle-même surmontée d'une couche imperméable percée d'un évidement, situé de telle sorte qu'il se trouve en face du méat urinaire lorsque la serviette est portée par la patiente.

2. Serviette absorbante selon la revendication 1, caractérisée en ce qu'il existe en outre une couche totalement imperméable sous la couche absorbante.

3.Serviette absorbante selon les revendications 1 ou 2,caractérisée en ce qu'elle comporte au-dessus de la couche imperméable une couche en tissu.

4.Serviette absorbante selon la revendication 3, caractérisée en ce que la couche est en tissu poreux,non hydrofugé.

5.Serviette absorbante selon l'une des revendications 1 à 4,caractérisée en ce que la couche absorbante est constituée de super-absorbants.

6.Serviette absorbante selon l'une des revendications 1 à 5, caractérisée en ce que le super-absorbant est du carboxyméthylcellulose de sodium insolubilisé,présenté en poudre noyé dans une pulpe ou emprisonné dans des feuilles.

7.Serviette absorbante selon l'une des revendications précédentes, caractérisée en ce que la couche supérieure et la couche imperméable sous-jacente forment une seule couche perforée,constituée d'un tissu composite,à base de tissu non tissé et plastique.

8.Serviette absorbante selon les revendications 3 et 5 à 7, caractérisée en ce que la couche supérieure en tissu non tissé, hydrofugé ou non,est perforée comme la couche imperméable sous-jacente.

9.Serviette absorbante selon l'une quelconque des revendications précédentes,caractérisée en ce que,sous la

0119919

11

couche imperméable , il existe une couche en tissu non tissé qui peut porter une partie adhésive pour fixation de la serviette au sous-vêtement.

10.Serviette absorbante selon l'une quelconque des revendications précédentes,caractérisée en ce que la couche non absorbante comprise entre la couche absorbante et la couche imperméable supérieure est en tissu non-tissé.

11.Serviette absorbante selon l'une quelconque des revendications précédentes, caractérisée en ce que les différentes couches sont solidaires sauf la couche absorbante.

12. Serviette absorbante selon l'une quelconque des revendications précédentes,caractérisée en ce que la couche non absorbante comprise entre la couche absorbante et la couche imperméable supérieure n'est pas solidaire des autres couches.

0119919

1/1

FIG.1

FIG.2

# Office européen des brevets

# RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| Y,D | BE-A- 893 697 (BEGHIN-SAY) * Page 5, ligne 20 - page 6, ligne 7; page 7, lignes 9-16, 30-32; figures 3,4,10,13 * | 1-6,10 | A 61 F 5/44 A 61 F 13/18 |
| A | | 7,11 | |
| | --- | | |
| Y | US-A-4 360 022 (USAMI et al.) * Colonne 2, lignes 13-20, 52-62; figure 5 * | 1-6,10 | |
| A | | 12 | |
| | --- | | |
| A,D | US-A-3 665 920 (DAVIS) * Colonne 3, lignes 24-27; figure 3 * | 1 | |
| | | | **DOMAINES TECHNIQUES RECHERCHES (Int. Cl. ³)** |
| | --- | | |
| A | US-A-3 805 790 (KACZMARZYK et al.) * Colonne 3, lignes 10-22; figures * | 1,9 | A 61 F |
| | --- | | |
| A | US-A-4 072 151 (LEVINE) * Colonne 3, lignes 26-31 * | 1-4 | |
| | --- | | |
| A | US-A-4 077 410 (BUTTERWORTH et al.) * Colonne 6, lignes 4-20; colonne 4, lignes 44-47; figure 3 * | 1,2,11 | |
| | --- -/- | | |

Le présent rapport de recherche a été établi pour toutes les revendications

| Lieu de la recherche | Date d'achèvement de la recherche | Examinateur |
|---|---|---|
| LA HAYE | 13-06-1984 | GLAS J. |

Office européen
des brevets

**RAPPORT DE RECHERCHE EUROPEENNE**

| DOCUMENTS CONSIDERES COMME PERTINENTS | | | Page  2 |
|---|---|---|---|
| Catégorie | Citation du document avec indication, en cas de besoin, des parties pertinentes | Revendication concernée | CLASSEMENT DE LA DEMANDE (Int. Cl. ³) |
| A | US-A-3 441 025  (RALPH)<br>* Colonne  1, lignes 25-29; fig-<br>ures * | 1 | |
| A | FR-A-1 170 636  (DU PONT DE<br>NEMOURS & CO.)<br>* Résumé, point A * | 1,5,6 | |
| | | | DOMAINES TECHNIQUES RECHERCHES (Int. Cl. ³) |

Le présent rapport de recherche a été établi pour toutes les revendications

| Lieu de la recherche<br>LA HAYE | Date d'achèvement de la recherche<br>13-06-1984 | Examinateur<br>GLAS J. |
|---|---|---|

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

& : membre de la même famille, document correspondant